# EUROPEAN PATENT APPLICATION

(11) **EP 2 246 049 A2**
(43) Date of publication of application: **03.11.2010**
(21) Application number: 10173114.9
(22) Date of filing: 24.05.2004
(51) Int. Cl.: A61K 31/355, A61K 31/56, C07J 53/00

(54) **Pharmaceutical composition and dosage forms for administration of hydrophobic drugs**

(30) Priority: 22.05.2003 US 444935
(62) Divisional of application: 04753162.9
(71) Applicant: Lipocine, Inc., Salt Lake City, UT 84103 (US)
(72) Inventor: Chen, Feng-Jing, Salt Lake City, UT 84111 (US); Patel, Mahesh V., Salt Lake City, UT 84124 (US); Fikstad, David T., Salt Lake City, UT 84102 (US); Zhang, Huiping, Salt Lake City, UT 844121 (US); Giliyar, Chandrashekar, Salt Lake City, UT 84102 (US)
(74) Representative: Walker, Ross Thomson

(57) **Abstract**

Pharmaceutical compositions and dosage forms for administration of hydrophobic drugs, particularly steroids, are provided. The pharmaceutical compositions include a therapeutically effective amount of a hydrophobic drug, preferably a steroid; a solubilizer, preferably a vitamin E substance; and a surfactant. The synergistic effect between the hydrophobic drug and the vitamin E substance results in a pharmaceutical formulation with improved dispersion of both the active agent and the solubilizer. As a result of the improved dispersion, the pharmaceutical composition has improved bioavailability upon administration. Methods of improving the bioavailability of hydrophobic drugs administered to a patient are also provided.

## Description

### REFERENCE TO RELATED APPLICATIONS

This application is a continuation-in-part of U.S. Patent Application No. 09/716,029, filed November 17, 2000, and a continuation-in-part of U.S. Patent Application No. 09/877,541, filed June 8, 2001, which is a continuation-in-part of U.S. Patent Application No. 09/345,615, filed June 30, 1999, and a continuation-in-part of U.S. Application No. 09/751,968, filed December 29, 2000, which is a continuation-in-part of U.S. Application No. 09/375,636, filed August 17, 1999, the disclosures of which are incorporated herein by reference in their entireties.

### TECHNICAL FIELD

The present invention relates generally to the delivery of hydrophobic drugs, such as steroids and benzoquinones. More specifically, the invention relates to novel pharmaceutical compositions in which a therapeutically effective amount of a hydrophobic active agent is combined with a vitamin E substance and a surfactant to form a uniform dispersion wherein the active agent is solubilized in the aqueous environment in a readily absorbable form.

### BACKGROUND

Numerous therapeutic agents are poorly soluble in aqueous medium and present difficult problems in formulating for effective administration to patients. Steroids in particular have very low water solubility and are useful therapeutic agents for a wide variety of medical conditions. Conventional formulations that incorporate these therapeutic agents suffer from several disadvantages such as incomplete or slow dissolution and/or highly variable dissolution profiles. Furthermore, following oral administration, these conventional formulations exhibit low and/or variable absorption. A well-designed formulation must, at minimum, be capable of presenting a therapeutically effective amount of the active substance to the desired absorption site, in an absorbable form.

A number of approaches are known for formulating therapeutic agents that are poorly soluble in water, for both oral and parenteral delivery.

One approach to improving the bioavailability of such active substances is to micronize the particles and to suspend them in a pharmaceutically acceptable matrix. For example, U.S. Patent Nos. 4,196,188; 4,963,540; and 5,140,021 disclose compositions for oral delivery of progesterone comprising micronized particles of crystalline progesterone in triglyceride vehicles. Such suspensions are difficult to manufacture, may be physically unstable, and may still suffer from poor dissolution and low and/or highly variable absorption. Similarly, compositions utilizing solid dispersions, such as the approach in FR 2, 647,346, which discloses a solid dispersion of the metastable progesterone II polymorph in a hydrophilic excipient, are difficult to manufacture consistently and may suffer from physical stability problems. Additionally this approach may still suffer from poor dissolution and low and/or highly variable absorption,

Another well-known approach uses surfactant micelles to solubilize and transport the therapeutic agent. Micelles, and pharmaceutical compositions containing micelles, have been extensively studies and are described in detail in the literature; see, *e.g*., Remington's Pharmaceutical Sciences, 17th ed. (1985). Although micellar formulations can solubilize a variety of hydrophobic therapeutic agents, the loading capacity of conventional micelle formulations is limited by the solubility of the therapeutic agent in the micelle surfactant. For many therapeutic agents, such solubility is too low to offer formulations that can deliver therapeutically effective doses.

Another approach is to solubilize the active substance in a triglyceride solvent, such as a digestible vegetable oil. For example, U.S. Patent No. 4,900,734 to Maxson et al. discloses a composition in which progesterone is dissolved in a highly unsaturated edible oil, These triglycerides are water insoluble themselves and do not normally disperse in aqueous environments such as the gastrointestinal tract. Typically, they must by emulsified by high shear or high temperature homogenization and stabilized with emulsifiers. In simplest form, a triglyceride-containing formulation suitable for delivering hydrophobic agents through an aqueous environment is an oil-in-water emulsion. The colloidal oil particles are relatively large and will often spontaneously agglomerate, eventually leading to complete phase separation. The large size slows the rate of transport of the colloidal particle and hence the rate of absorption of the therapeutic agent. Thus these triglyceride compositions are subject to a number of significant limitations and disadvantages, such as physical instability and lack of homogeneity, and are likely to suffer from poor and variable absorption. A further disadvantage of triglyceride-containing compositions is the dependence of the therapeutic agent absorption on the rate and extent of lipalysis (*e.g*. see WO 9524893 and WO 9740823).

Other solubilizers of particular utility for hydrophobic active agents are described in U.S. Patent Application No. 09/716,029 to Chen et al. The vitamin E substances disclosed therein include fatty acid esters of glycerol, such as mono-, di-, and triglycerides and acetylated mono- and diglycerides, and mixtures thereof, fatty acid esters of propylene glycol, such as mono- and di-fatty acid esters of glycerol and mixtures thereof, trialkyl citrate, glyccryl acetate and lower alcohol fatty acid esters.

WO 01/49262; U.S. Patent No. 6,458,373; and U.S. Patent No. 6,193,985 disclose the use of solubilizers that require high levels of hydrophilic surfactants, high shear, or high temperature homogenization to disperse the solubilizers sufficiently to form even a coarse dispersion in an adequate medium. Formation of a fine dispersion, which would make an effective carrier for oral delivery of the active agent, is often difficult or impossible to achieve. As with the triglyceride emulsions, these can be difficult to manufacture and/or unstable on storage, and may lead to poor and variable absorption.

Thus, there is a need for pharmaceutical compositions for the delivery of therapeutic levels of active agents that overcome the solubility, physical stability, and absorption limitations of conventional approaches using micronization, emulsification, or solubilization,

### SUMMARY OF THE INVENTION

In the present invention, we have found an unexpected synergism between an active agent and a solubilizer. We have found that for certain therapeutic actives, the active agent has a critical role in improving the dispersion of the solubilizer upon dilution in an aqueous media, allowing for dispersion of much higher levels of both solubilizer and active agent in the aqueous environment. In particular, such synergism can be exemplified by compositions comprising an active agent, a vitamin E substance as the solubilizer, and a surfactant as a dispersing aid, wherein the presence of the active agent improves the dispersion of the solubilizer and thus further increases the amount of active agent which can be dispersed in a readily absorbable form. This unexpected synergism between the active agent, a vitamin E substance, and a surfactant allows for very high drug loading as well as excellent dispersion, keeping the drug substantially solubilized upon dilution in an aqueous environment such as the gastrointestinal tract in a finely dispersed phase that is optimal for absorption.

Accordingly, it is a primary object of the invention to address the above-mentioned need in the art by providing a pharmaceutical composition and dosage form for orally administering therapeutic agents.

In a first embodiment of the present invention, a pharmaceutical composition is provided comprising a therapeutically effective amount of an active agent, a vitamin E substance and a surfactant, wherein upon dilution of the composition, the active agent increases the extent of dispersion of the vitamin E substance by at least 20% relative to the dispersion of the composition without the active agent.

In a second embodiment of the present invention, a pharmaceutical composition is provided comprising a therapeutically effective amount of an active agent, a vitamin E substance and a surfactant, wherein after a 100X dilution of the composition in an aqueous medium, at least 30% of the active agent or the vitamin E substance is dispersed in an aqueous phase.

The present invention also encompasses methods of improving the bioavailability of active agents, and steroids in particular, in patients through the administration of the claimed pharmaceutical compositions in suitable dosage forms.

### DETAILED DESCRIPTION OF THE INVENTION

**I. PHARMACEUTICAL COMPOSITIONS**

The present invention overcomes the problems associated with the conventional approaches for preparing formulations containing hydrophobic active agents by providing unique pharmaceutical compositions comprising a therapeutically effective amount of an active agent, a solubilizer and, optionally, a dispersing aid, that are more readily dispersed upon mixing with an aqueous medium than those which would be obtained without the particular combination of solubilizer and active agent.

Surprisingly, the present inventors have found that with a composition of an active agent, such as a steroid or benzoquinone; a solubilizer, such as a vitamin E substance; and a dispersion aid, such as a surfactant, a synergistic combination results wherein upon dilution in aqueous media at an appropriate dilution factor the dispersion of both the active agent and the solubilizer is improved and thus the active agent is solubilized in the aqueous environment in a readily absorbable form. A synergistic combination of an appropriate active agent and solubilizer is observed, such that the presence of the active agent improves the dispersion of the solubilizer (i.e. increases the amount of solubilizer which may be dispersed) and thus further increases the amount of active agent which can be dispersed in a readily absorbable form.

Within the context of the present invention, the term "dispersion" is used to refer to the extent to which the composition, in particular the active agent and the solubilizer, are uniformly distributed in the aqueous phase after dilution in an aqueous medium, such as water, simulated gastric fluid, or simulated intestinal fluid. In general, it is expected that aqueous dispersion of the active agent is critical for oral absorption. The extent of dispersion of the composition can be indirectly measured by diluting the composition in an aqueous medium at a selected dilution factor, preferably 10X to 1000X, most preferably 100X; gently mixing the dilution for a physiologically realistic duration, sampling from the aqueous phase; and assaying for either active agent or the solubilizer. The extent of dispersion is then defined as the fraction of the total drug or solubilizer which is distributed in the aqueous phase and thus readily available for absorption. The undispersed fraction is the fraction of the total drug or solubilizer would then typically be present in separate oil or solid layers and non-uniformly distributed large globules, or large aggregates of particulates which would be then unavailable for absorption. The characteristics of the dispersion can be further assessed by separating out larger particles or globules by filtration or centrifugation, then assaying for either the active agent or the solubilizer (e.g. vitamin E) or both in the filtrate or supernatant.

In a preferred embodiment, the composition forms a "fine dispersion" in which the composition is dispersed such that at least 30% of the active agent or vitamin E substance solubilizer is in particles which will pass through a filter with 0.45 µ nominal pore size.

As a general rule, it is expected that aqueous dispersion of the active agent is critical for absorption and that the more finely dispersed the active agent is, the more effectively it will be absorbed. Other techniques for characterizing the effectiveness of the dispersion may also be used, such as filtration of the aqueous dispersion with varying nominal pore size and demonstrating an increase in the fraction of active agent or solubilizer in the filtrate of any given size, or centrifugation to demonstrate an increase in the fraction of active agent or solubilizer in aqueous layer. A similar comparison may be made based on measuring the volume-weighted particle size distribution by photon correlation spectroscopy (dynamic laser light scattering) and showing an increase in the fraction of particles with particle diameter below a certain threshold, a decrease in the fraction of particles with diameter above a certain threshold, or a reduction in the volume-weighted mean particle size. Alternatively, an increase in the effectiveness of the dispersion may be shown by a reduction in the absorbance of light by an aqueous dilution at visual wavelengths (e.g. 400 nm).

In a preferred first embodiment of the present invention, a pharmaceutical composition is provided comprising a therapeutically effective amount of an active agent, a vitamin E substance and a surfactant, wherein upon dilution of the composition, the active agent increases the extent of dispersion of the vitamin E substance by at least 20% relative to the dispersion of the composition without the active agent.

In a preferred second embodiment of the present invention, a pharmaceutical composition is provided comprising a therapeutically effective amount of an active agent, a vitamin E substance and a surfactant, wherein after a 100X dilution of the composition in an aqueous medium, at least 30% of the active agent or the vitamin E substance is dispersed in an aqueous phase.

In another embodiment, a pharmaceutical composition is provided comprising a therapeutically effective amount of an active agent, a solubilizer and, optionally, a dispersing aid, wherein the amount of active agent improves the dispersion of the solubilizer over that which would be achieved with the same solubilizer without the active agent upon contact with an aqueous medium.

In yet another embodiment, a pharmaceutical composition is provided comprising a therapeutically effective amount of an active agent, a solubilizer and a dispersing aid, wherein the solubilizer is present in an amount such that more of the active agent is dispersed in aqueous medium than that which would be achieved with the same active agent and dispersing aid without the solubilizer.

In still another embodiment, a pharmaceutical composition is provided comprising a therapeutically effective amount of an active agent, a solubilizer and a dispersing aid, wherein the active agent is present in an amount such that at least 30% of the active agent and/or the solubilizer present in the composition is dispersed upon dilution with an aqueous medium.

In a further embodiment, a pharmaceutical composition is provided comprising a therapeutically effective amount of an active agent, a solubilizer and a dispersing aid, wherein the active agent and the solubilizer are present in amounts such that the composition forms a more effective aqueous dispersion than that which would be achieved without the active agent.

In the embodiments set forth above, where applicable, the improvement of the dispersion of either the active agent or the solubilizer or the improvement in the effectiveness of the dispersion is on the order of at least 20%, preferably at least 30%, more preferably at least 50%, and the dispersion of the active agent or the solubilizer is at least 30%, with a dispersion of at least 50% preferred, a fine dispersion of at least 30% more preferred, and a fine dispersion of at least 50% most preferred.

In another embodiment, a pharmaceutical composition is provided comprising a therapeutically effective amount of an active agent, a solubilizer, and optionally, a dispersing aid, wherein the active agent is present in an amount of from about 0.1 to 30 % w/w of the composition; the solubilizer in the composition is present in an amount of from about 1 to 99 % w/w of the composition; and the dispersing aid is present in an amount from about 1 to 99% of the composition

Preferably, the concentrations of each of the active agent, solubilizer, and surfactant of the claimed pharmaceutical composition will have the following ranges: active agent from 0.01% to 30% w/w; solubilizer (vitamin E substance) from 1-95% w/w; and surfactant from 5-85% w/w. The concentrations of some exemplary steroids are provided as follows: progesterone - 1-300 mg/dosage form (0.1% to 30% w/w); testosterone - 10 mg to 300 mg/dosage form ( at least 1% w/w); and DHEA - 50 to 300 mg/dosage form (at least 5% w/w).

Tables 1-2, 2-2, 3-2, 4-2, 5-2, 6-3, 7-2, 8-3 and 9-2 from Examples 1-9 show that the synergy between the active agents and the vitamin E substances results in a pharmaceutical composition with a very high percent of dispersion of the active agent and/or the vitamin E substance solubilizer. Table 1-2 shows that as the concentration of active agent is increased from 0% to 15%, the dispersion of both the active agent and the vitamin E substance increase. Table 8-3 also shows that the careful selection of a solvent or cosolubilizer may further increase the dispersion of the composition.

Examples 10-25 set forth exemplary compounds that fall within the scope of the pharmaceutical compositions of the present invention.

**A. ACTIVE AGENTS**

The active agent of the present invention is characterized by the fact that it is solubilized in aqueous dispersion by the solubilizer and has a synergistic role in improving the dispersibility of the solubilizer (and consequently of the active agent itself) upon dilution in aqueous media. The active agent can be said to "improve" the dispersibility of the solubilizer if it is present at levels such that at the selected dilution factor it increases the extent of dispersion of the solubilizer by at least about 20% relative to the same composition without the active agent. In one embodiment of the present invention, a pharmaceutical composition is provided comprising a therapeutically effective amount of an active agent, a solubilizer and, optionally, a dispersing aid, wherein upon dilution of the composition, the active agent is present in an amount to increase dispersion of the solubilizer by at least 20% more than that winch would be achieved with the same composition without the active agent.

Preferably, the active agent is present such that after a 100X dilution of the composition the active agent is at least 30% dispersed in the aqueous phase, with an active agent dispersion of at least 50% being preferred. More preferably, the active agent is present such that as least 30% of the drug is in fine dispersion. Most preferably the active agent is present such that at least 50% of the drug is in a fine dispersion.

While this approach may be broadly applicable to many classes of active agents, particularly hydrophobic actives, we have found that drugs in the class of steroids and benzoquinones are particularly effective in this regard.

The following lists set forth exemplary active agents for use in the present invention; those of ordinary skill in the art will readily recognize that suitable active agents may be used in the present invention either alone or in combination.

Steroids are compounds based on the cyclopenta[*a*]phenanthrene structure. Examples of steroids which have been shown to be suitable for the current invention include those with the androstane structure. Examples of such androstane steroids include cetadiol, clostebol, danazol, dehydroepiandrosterone (DHEA) (also, prasterone or dehydroisoandrosterone), DHEA sulfate, dianabol, dutasteride, exemestane, fmasteride, nerobol, oxymetholone, stanolone, stanozolol, testosterone, 17-alpha-methyltestosterone, and methyltestosterone enanthate.

Another group steroids, which have been shown to be suitable, are those based on the cholane or cholesterol structure. Examples of such steroids are brassicasterol, campesterol, chenodeoxycholic acid, clionasterol, desmosterol, lanosterol, poriferasterol, β-sitosterol, stigmasterol, and ursodeoxycholic acid.

Another suitable class of steroids for use in the present invention are those steroids based on the estrane structure. Examples of such estranes include desogestrel, equilin, 17-alpha-dihydroequilin, 17-beta-dihydroequilin, 17-alpha-estradiol, 17-beta-estradiol (estradiol), ethinyl estradiol, estriol, estrone, levonorgestrel, lynestrenol, mestranol, mibolerone, mifegyne, mifepristone, nandrolone, norethindrone (or noretliistrone), norethindrone acetate (or norethisterone acetate), nortestosterone.

Also suitable is the steroid class based on the pregnane structure. Examples of such pregnanes include alfaxalone, beclomethasone, budesonide, clobetasol, clobetasone, corticosterone, desoxycorticosterone, cortisol, cortisone, dihydrocortisone, cyproterone, desonide, dexamethasone, eplerenone, epoxypregnenolone, flumethasone, megestrol, melengestrol, prednisolone, prednisone, pregnanediol, pregnanolone, pregnenolone, allopregnanolone, epiallopregnanolone, progesterone, medroxyprogesterone, spironolactone, and tibolone.

It is to be understood that steroids suitable for the present invention arc not limited to those disclosed herein and include any secondary steroids, such as for example, vitamin D.

Steroid esters, such as the acetate, benzoate, cypionate, decanoate, enanthate, hemisuccinate, hexahydrobenzoate, 4-methylvalerate, propionate, stearate, valerate, and undecanoate esters would also be suitable for the present invention.

Examples of suitable benzoquinones include ubiquinones, such as coenzyme Q10, embelin, idebenone [2,3-dimethoxy-5-methyl-6-(10-hydroxydecyl)-1,4-benzaquinanc], pyrroloquinoline quinone, and seratrodast [7-(3,5,6-trimethyl-1,4-benzoquinon-2-yl)-7-phenylheptanoic acid].

Examples of other active agents which may be suitable for this invention include, without limitation: abecarnil, acamprostate, acavir, acebutolol, aceclofenac, acemetacin, acetaminophen, acetaminosalol, acetanilide, acetohexamide, acetophenazine maleate, acetophenazine, acetoxolone, acetoxypregnenolone, acetretin, acrisorcin, acrivastine, acyclovir, adinazolam, adiphenine hydrochloride, adrafinil, adrenolone, agatroban, ahnitrine, akatinol, alatrofloxacin, albendazole, albuterol, aldioxa, alendronate, alfentanil, alibendol, alitretinoin, allopurinol, allylamines, allylestrenol, alminoprofen, almotriptan, alosetron, aloxiprin, alprazolam, alprenolol, amantadine, ambucetamide, amidephrine, amidinomycin, amiloride, aminoarylcarboxylic acid derivatives, aminoglutethimide, aminoglycosides, aminopentamide, aminopromazine, aminorex, amiodarone, amiphenazole, amiprilose, amisulpride, amitriptyline, amlexanox, amlodipine, amodiaquine, amosulalol, amotriphene, amoxapine, amoxicillin, amphecloral, amphetamine, amphomycin, amphotericin, ampicillin, ampiroxicam, amprenavir, anmnone, amsacrine, amyl nitrate, amylobarbitone, anagestone acetate, anastrozole, andinocillin, androstenediol, androstenediol-17-acetate, androstenediol-17-benoate, androstenediol-3-acetate, androstenediol-3-acetate-17-benzoate, androstenedione, androsterone acetate, androsterone benzoate, androsterone propionate, androsterone, angiotensin, anidulafungin, aniracetam, apazone, apicycline, apoatropine, apomorphine, apraclonidine, aprepitant, aprotinin, arbaprostil, ardeparin, aripiprazole, arnikacin, arotinolol, arstiinol, arylacetic acid derivatives, arylalkylamines, arylbutyric acid derivatives, arylcarboxylic acids, arylpiperazines, arylpropionic acid derivatives, aspirin, astemizole, atenolol, atomoxetine, atorvastatin, atovaquone, atropine, auranofin, azapropazone, azathioprine, azelastine, azetazolamide, azithromyein, baclofen, bambuterol, bamethan, barbitone, barnidipine, basalazide, beclamide, beclobrate, befimolol, bemegride, benazepril, bencyclane, bendarac, bendazol, bendroflumethiazide, benethamme penicillin, benexate hydrochloride, benfurodil hemisuccinate, benidipine, benorylate, bentazepam, benzhexol, benziodarone, benznidazole, benzoctamine, benzodiazepine derivatives, benzodiazepine, benzonatate, benzphetamine, benzylmorphine, beperiden, bephenium hydroxynaphthoate, bepridil, betahistine, betamethasone, betaxolol, bevantolol, bevonium methyl sulfate, bexarotene, bezadoxifine, bezafibrate, bialamicol, biapenem, bicalutamide, bietamiverine, bifonazole, binedaline, binifibrate, biricodar, bisacodyl, bisantrene, bisoprolol, bitolterol, bopindolol, boswellic acid, bradykinin, bretylium, bromazepam, bramocriptine, bromperidol, brotizolam, brovincamine, buciclate, bucloxic acid, bucumolol, budralazine, bufeniode, bufetolol, buflomedil, bufuralol, bumetanide, bunitrolol, bupranolol, buprenorphine, buproprion, buspirone, busulfan, butalamine, butarphenol, butaverine, butenafine, butenafme, butidrine hydrochloride, butobarbitone, butoconazole nitrate, butoconazole, buiofilol, butorphenol, butropium bromide, cabergoline, calcifediol, calcipotriene, caicitriol, caldiribine, cambendazole, camioxirole, camostat, camposterol, camptothecin, candesartan, candoxatril, capecitabine, caprate, capsaicin, captopril, carazolol, carbacephems, carbamates, carbamezepine, carbapenems, carbarsone, carbatrol, carbenoxolone, carbimazole, carbromal, carbuterol, carisoprodol, carotenes, caroverine, carteolol, carvedilol, cefaclor, cefazolin, cefbuperazone, cefepime, cefoselis, ceftibuten, celcoxib, celecoxib, celiprolol, cephaeline, cephalosporin C, cephalosporins, cephamycins, cerivastatin, certoparin, cetamolol, cetiedil, cetirizine, cetraxate, chloracizine, chlorambucil, chlorbetamide, chlordantoin, chlordiazepoxide, chlormadinone acetate, chlormethiazole, chloroquine, chlorothiazide, chlorpheniramine, chlorphenoxamide, chlorphentermine, chlorproguanil, chlorpromazine, chlorpropamide, chlorprothixene, chlortetracycline, chlorthalidone, cholecalciferol, chromonar, ciclesonide, ciclonicatc, cidofivir, eiglitazone, cilansetron, cilostazol, cimetidine, cimetropium bromide, cinepazet maleate, einnamedrine, cinnarizine, cinolazepam, cinoxacin, eiprofibrate, ciprofloxacin, cisapride, cisplatin, citalopram, citicoline, clarithromycin, clebopride, clemastine, clenbuterol, clidanac, clinofibrate, clioquinol, clobazam, clobenfurol, clobenzorex, clofazimine, clofibrate, clofibrie acid, cloforex, clomipramine, clonazepam, clonidine, clonitrate, clopidogrel, clopirac indomethacin, cloranolol, cloricromen, clorprenaline, clortermine, clotiazepam, clotrimazole, cloxacillin, clozapine, cmepazide, codeine methyl bromide, codeine phosphate, codeine sulfate, codeine, colloidal bismuth subcitrate, cromafiban, cromolyn, cropropamide, crotethamide, curcumin, cyclandelate, cyclarbamate, cyclazocine, cyelexedrine, cyclizine, cyclobenzaprine, cyclodrine, cyclonium iodide, cyclopentamine, cyclosporin, cypionate, cyproheptadine, cyproterone acetate, cytarabine, dacarbazine, dalfopristine, dantrolene sodium, dapiprazole, darodipine, decanoate, decitabine, decoquinate, dehydroemetine, delavirdine, delaviridine, demeclocycline, denopamine, deramciclone, descitalopram, desipramine, desloratadine, 3-ketodesogestrel, desomorphine, desoxymethasone, detomidine, dexamphetamine, dexanabinol, dexchlorpheniramine, dexfenfluramine, dexmethylphenidate, dexrazoxane, dextroamphetamine sulfate, dextroamphetamine, dextropropoxyphene, DHEA, diacetate, diamorphine, diazemine, diazepam, diaziquinone, diazoxide, dibromopropamidine, dichlorophen, diclofenac, dicoumarol, didanosine, dideoxyadenosine, diethylpropion, difemerine, difenamizole, diflunisal, digitoxin, digoxin, dihidroergotamine, dihydrocodeine, dihydrocodeinone enol acetate, dihydroergotamine mesylate, dihydroergotamine, dihydrogesterone, dihydromorphine, dihydropyridine derivatives, dihydrostreptomyein, dihydrotachysterol, dihydroxyaluminum acetylsalicylate, diiodohydroxyquinoline, diisopromine, dilazep, dilevalol, dilitazem, diloxanide furoate, diloxanide, diltiazem, dimefline, dimenhydrinate, dimethisterone, dimetofrine, dimorpholamine, dinitolmide,
dioxaphetyl butyrate, dioxethedrine, diphemethoxidine, diphenhydramine, diphenoxylate, diphetarsone, dipivefrin, diponium bromide, dipyridamole, dirithromycin, disopyramide, divalproex sodium, dofetilide, domperidone, donezepil, dopexamine, dopradil, dosmalfate, doxapram, doxazosin, doxefazepam, doxepin, doxycycline, drofenine, dromostanolone propionate, dromostanolone, dronabinol, droperidol, droprenilamine, d-threo-methylphenidate, duloxetine, ebrotidine, eburnamonine, ecabet, ecenofloxaein, econazole nitrate, edavarone, edoxudine, efavirenz, effivarenz, efloxate, eledoisin, eletriptan, elgodipine, ellipticine, emepronium bromide, emetine, enalapril, enanthate, encainide, enlopitat, enoximone, enprostil, entacapone, epanolol, ephedrine, epinastine, epinephrine, epirubicin, epleronone, eposartan, ergocalciferol, ergoloid mesylates, ergotamine, ertapenum, erythromycin, erytlirityl tetranitrate, esaprazole, escitalopram, esmolol, esomeprazole, esonarimod, estazolam, estradiol benzoate, estramustine, estriol succinate, estrone acetate, estrone sulfate, etafedrine, etafenone, ethacrynic acid, ethamivan, ethinamate, ethinylestradiol 3-acetate, ethinylestradiol 3-benzoate, ethinylestradiol, ethionamide, ethisterone (17α-ethinyltestosterone), ethoproparine, ethotoin, ethoxyphenamine, ethylestrenol, ethylmorphine, ethylnorepinephrine, ethynodiol diacetate, etodolac, etofibrate, etolzoside, etoricoxib, etretinate, everolimus, exalamide, examestane, examorelin, ezemitibe, falecalcitriol, famcielovir, famotidine, fantofarone, farapenum, farglitazar, fasudil, felbamate, felodipine, fenalamide, fenbufen, fenbutrazate, fendiline, fenfluramine, fenoldopam, fenoprofen, fenoterol, fenoverine, fenoxazoline, fenoxedil, fenpiprane, fenproporex, fenspiride, fentanyl, fexofenadine, flavoxate, flecainide, flopropione, floredil, floxuridine, fluconazole, flucytosine, fludarabine, fludiazepam, fludrocortisone, flufenamic acid, flunanisone, flunarizine, flunisolide, flunitrazepam, fluocortolone, fluoxetine, flupenthixol decanoate, fluphenazine decanoate, fluphenazine enanthate, fluphenazine, fluproquazone, flurazepam, flurbiprofen, flurogestone acetate, fluticasone propionate, fluvastatin, fluvoxamine, fominoben, formoterol, foscarnet, foscarnet, fosinopril, fosphenytoin, frovatirptan, fudosteine, fumagillin, furazolidone, furazolidone, furfurylmethyl amphetamine, furosemide, gabapentin, gabexate, gaboxadol, galanthamine, gallopamil, gammaparin, ganciclovir, ganglefene, gefarnate, gemcitabine, gemfibrozil, gepirone, gestadene, ghrelin, glatiramer, glaucarubin, glibenclamide, gliclazide, glimepiride, glipizide, gluconic acid, glutamicacid, glyburide, glyceryl trinitrate, glymepiride, granisetron, grepafloxacin, griseofulvin, guaiazulene, guanabenz, guanfacine, halofantrine, haloperidol decanoate, haloperidol, haloxazolam, hepronicate, heptanoate, hexobendine, hexoprenaline, hydramitrazine, hydrazides, hydrochlorothiazide, hydrocodone, hydrocortisone, hydromorphone, hydroxyamphetamine, hydroxymethylprogesterone acetate, hydroxymethylprogesterone, hydroxyprogesterone acetate, hydroxyprogesterone caproate, hydroxyprogesterone, hymecromone, hyoscyamine, ibopamine, ibudilast, ibufenac, ibuprofen, ibutilide, idoxuridine, ifenprodil, igmesine, iloprost, imatinib, imidapril, imidazoles, imipenem, imipramine, imolamine, incadronic acid pergolide, indanazoline, indenolol, indinavir, indomethacin, indoramin, inosinepranobex, inositol niacinate, iodoquinol, ipidracine, iproniazid, irbesartan, irinotecan, irsogladine, isobutyrate, isocaprate esters, isoetharine, isometheptene, isoproterenol, isosorbide dinitrate, isosorbide mononitrate, isosorbide dinitrate, isoxsuprine, isradipine, itasetron, itraconazole, itramintosylate, ivermectin, kallidin, kallikrein, kanamycin, ketamine, ketoconazole, ketoprofen, ketorolae, ketotifen, labetalol, lafutidine, lamifiban, lanivudine, lamotrigine, lanatoside c, lansoprazole, lasofoxifene, leflunomide, leminoprazole, lercanadipine, lesopitron, letrozole, leucovorin, levalbuterol, levallorphan, levetiracetam, levetriacetam, levobunolol, levodopa, levofloxacin, levophacetoperane, levorphanol, lidocaine, lidoflazine, lifibrol, limaprost, linezolid, lintitript, liranaftate, lisinopril, lisuride, lobeline, lobucavir, lodoxamide, lomefloxacin, lomerizine, lomustine, loperamide, lopinavir, loprazolam, loracarbef, loratadine, lorazepam, lorefloxacin, lormetazepam, losartan, lovasatain, lovastatin, loxapine succinate, loxapine, 1-threo-methylphenidate, lumiracoxib, lysine acetylsalicylate, lysozyme, lysuride, mabuterol, mafenide, magnesium acetylsalicylate, malgramostin, mannitol hexanitrate, maprotiline, mazindol, mebendazole, meclizine, meclofenamic acid, mecloxaminepentapiperide, medazcpam, medibazine, medigoxin, medrogestone, medroxyprogesterone acetate, mefenamic acid, niefenorex, mefloquin, mefloqume, megestrol acetate, melengestrol acetate, melphalan, mematine, mepenzolate bromide, meperidine, mephenoxalone, mephentennine, mepindolol, mepixanox, meprobamate, meptazinol, mercaptopurine, merropenum, mesalamine, mesalazinc, mesoridazine besylate, mesoridazine, metaclazepam, metamfepramone, metampicillin, metaproterenol, metaraminol, methacycline, methadone hydrochloride, methadone, methamphetamine, methaqualone, metharnphetamine, methoin, methotrexate, methoxamine, methsuximide, methylhexaneamine, methylphenidate d-threo-methylphenidate, methylphenidate, mcthylphetiobarbitone, methylprednisolone, methysergide, metiazinic acid, motizoline, metoclopramide, metolazone, metoprolol, metoxalone, metripranolol, metronidazole, inexiletine, mexilitene, metaxalone, mianserin, mibefradil, miconazole, midazolam, midodrine, miglitol, milnacipran, milrinone, minoxidil, mirtazapine, misoprostol, mitomycin, mitotane, mitoxantronc, mizolastine, modafinil, mofebutazone, mofetil, molindone hydrachloride, molindone, molsidomine, monatepil, montelukast, monteplase, moprolol, moricizine, morphine hydrochloride, morphine sulfate, morphine, morpholine salicylate, mosapramine, moxifloxacin, moxisylvyte, moxonidine, mycophenolate, nabumetone, nadolol, nadoxolol, nadroparin, nafamostat, nafronyl, naftopidil, nalbuphine, nalidixic acid, nalmefene, nalorphine, naloxone, naltrexone, nandrolone benzoate, nandrolone cyclohexanecarboxylate, nandrolone cyclohexane-propionate, nandrolone decanoate, nandrolone furylpropionate, nandrolonc phenpropionate, naphazoline, naproxen, naratriptan, natamycin, nateglinide, nebivalol, nedocromil, nefazodone, nefopam, nelfinavir, nemonapride, neomycin undecylenate, neomycin, neotrofin, nesiritide, n-ethylamphetamine, nevibulol, nevirapine, nexopamil, nicametatc, nicardipine, nicergoline, nicofibrate, nicofuranose, nicomorphine, nicorandil, nicotinyl alcohol, nicoumalone, nifedipine, nifenalol, nikethamide, nilutamide, nilvadipine, nimodipine, nimorazole, nipradilol, nisoldipine, nitisonone, nitrazepam, nitrofurantoin, nitrofurazone, nitroglycerin, nizatidine, norastemizole, norepinephrine, norethynodrel, norfenefrine, norfloxacin, norgestimate, norgestrel, norgestrienone, normethadone, noiniethisterone, normorphine, norpseudoephedrine, nortriptyline, novantrone, nylidrin, nystatin, octamylamine, octodrine, octopamine, ofloxacin, olanzapine, olanzapine, olapatadine, olmesartan, olopatidine, olsalazine, omapatrilat, omeprazole, ondasetron, opium, oprevelkin, orlistat, ornidazole, ornoprostil, oseltamivir, oxaliplatin, oxamniquine, oxandrolone, oxantel embonate, oxaprozin, oxatomide pemirolast, oxatomide, oxazepam, oxcarbazepine, oxfendazole, oxiconazole, oxiracetam, oxolinicacid, oxprenolol, oxycodone, oxyfedrine, oxymetazoline, oxymorphone, oxyphenbutazone, oxyphencyclimine, oxyprenolol, ozagrel, paclitaxel, palonosctron, pantoprazole, papaverine, paracalcitol, paramethadione, parecoxib, pariprazole, paromomycin, paroxetine, parsalmide, pazinaclone, pemoline, penbutolol, pencielovir, penicillin G benzathine, penicillin G procaine, penicillin V, penicillins, pentaerythritol tetranitrate, pentaerythritol tetranitrate, pentapiperide, pentazocine, pentifylline, pentigetide, pentobarbitone, pentorex, pentoxifylline, pentrinitrol, perbuterol, perenzepine, pergolide, perhexiline, perindopril erbumine, perospirone, perphenazine pimozide, perphenazine, phanquinoue, phenacemide, phenacetin, phenazopyridine, phencarbamide, phendimetrazine, phenelzine, phenindione, phenmetrazine, phenobarbitone, phenoperidine, phenothiazines, phenoxybenzamine, phensuximide, phentermine, phentolamine, phenyl salicylate, phenylacetate, phenylbutazone, phenylephrinehydrochloride, phenylpropanolamine hydrochloride, phenylpropanolaminehydrochloride, phenylpropyl-methylamine, phenytoin, phloroglucinol, pholedrine, physostigmine salicylate, physostigmine, phytonadiol, phytosterols, piapenum, picilorex, piclamilast, picrotoxin, picumast, pifarnine, pilsicainide, pimagedine, pimeclone, pimecrolimus, pimefylline, pimozide, pinaverium bromide, pindolol, pioglitazone, pipcracillin, piperazine estrone sulfate, piperazine derivatives, piperilate, piracetam, pirbuterol, pirenzepine, piribedil, pirifibrate, piroxicam, pitavastatin, pizotyline, plaunotol, polaprezinc, polybenzarsol, polyestrol phosphate, practolol, pralnacasan, pramipexole, pranlukast, pravastatin, prazepam, praziquantel, prazosin, pregabalin, prenalterol, prenylamine, pridinol, prifinium bromide, primidone, primipramine, probenecid, probucol, procainamide, procarbazine, procaterol, prochlorperazine, proguanil, pronethalol, propafenone, propamidine, propatyl nitrate, propentoffyline, propionate, propiram, propoxyphene, propranolol, propylhexedrine, propylthiouracil, protokylol, protriptyline, proxazole, pseudoephedrine, purines, pyrantel embonate, pyrazoles, pyrazolones, pyridofylline, pyrimethamine, pyrimidines, pyrrolidones, quazepam, quetiapine, quetuapine, quinagolide, quinapril, quinestrol, quinfamide, quinidine, quinine sulfate, quinolones, quinupritin, rabalzotan, rabeprazole sodium, rabeprazole, racefimine, ramatroban, ramipril, ranitidine, ranolazine, ransoprazole, rasagiline, rcbamipide, refludan, repaglinide, repinotan, repirinast, reproterol, reserpine, retinoids, ribavirin, rifabutine, rifampicin, rifapentine, rilmenidine, riluzole, rimantadine, rimiterol, rioprostil, risperidone, ritanovir, ritapentine, ritipenem, ritodrine, ritonavir, rivastigmine, rizatriptan, rociverine, rofeocoxib, rohypnol, rolipram, romoxipride, ronifibrate, ropinirole, ropivacaine, rosaprostol, rosiglitazone, rosuvastatin, rotinolol, rotraxate, roxatidine acetate, roxindole, rubitecan, salacetamide, salicin, salicylamide, salicylic acid derivatives, salmeterol, saquinavir, saquinavir, scopolamine, secnidazole, selegiline, semotiadil, sertindole, sertraline, sibutramine, sildenafil, simfibrate, simvastatin, siramesine, sirolimus, sitaxsentan, sofalcone, somotiadil, sorivudine, sotalol, soterenol, sparfloxacin, spasmolytol, spectinomycin, spiramycin, spizofurone, stavudine, streptomycin, succinylsulfathiazole, sucralfate, sufentanil, sulconazole nitrate, sulfacetamide, sulfadiazine, sulfaloxicacid, sulfarside, sulfmalol, sulindae, suloctidil, sulphabenzamide, sulphacetamide, sulphadiazine, sulphadoxine, sulphafurazole, sulphamerazme, sulphamethoxazole, sulphapyridine, sulphasalazine, sulphinpyrazone, sulpiride, sulthiame, sultopride, sultroponium, sumanirole, sumatriptan, sunepitron, superoxide dismuiase, suplatast, suramin sodium, synephrine, tacrine, tacrolimus, tacrolimus, tadalafil, talinolol, talipexole, tamoxifen, tamsulosin, targretin, tazanolast, tazarotene, tazobactum, tecastimezole, teclozan, tedisamil, tegaserod, telenzepine, telmisartan, temazepam, teniposide, teprenone, terazasin, terbenafine, terbinafine, terbutaline sulfate, terbutaline, terconazole, terfenadine, terodiline, terofenamate, tertatolol, testolactone, 4-dibydrotestosterone, tetracyclics, tetracycline, tetrahydrocannabinol, tetrahydrozoline, thalidomide, theofibrate, thiabendazole, thiazinecarboxamides, thiocarbamates, thiocarbamizine, thiocarbarsone, thioridazine, thiothixene, tiagabine, tiamenidine, tianeptine, tiaprofenic acid, tiaramide, ticiopidine, tigloidine, tilisolol, timolol, tinidazole, tinofedrine, tinzaparin, tioconazole, tipranavir, tirapazamine, tirofiban, tiropramide, titanicene, tizanadine, tizanidine, tizinadine, tocainide, tolazamide, tolazoline, tolbutamide, tolcapone, tolciclate, tolfenamic acid, toliprolol, tolteridine, tolterodine, tonaberstat, topiramate, topotecan, torasemide, taremifene citrate, toremifene, tosufloxacin, tramadol, tramazoline, trandolapril, tranilast, tranylcypromine, trapidil, traxanox, trazodone, tretoquinol, triacetin, triamcinolone, triampterine, triamterine, triazolam, triazoles, tricromyl, tricyclics, trifluoperazine hydrochloride, trifluoperazine, triflupromazine, trifluridine, trihexyphenidyl hydrochloride, trihexyphenidyl, trimazosin, trimebutine, trimetazidine, trimethoprim, trimgestone, trimipramine, trimoprostil, trithiozine, troglitazone, trolnitrate phosphate, tromethamine, tropicamide, trovafloxacin, troxipide, tuaminoheptane, tulobuterol, tymazoline, tyramine, undecanoate, undecanoic acid, urinastatin, valacyclovir, valdecoxib, valerate, valganciclovir, valproic acid, valsartan, vancomycin, vardenafil, venlafaxine, venorelbine, verapamil, verapimil, vidarabine, vigabatrin, vincamine, vinpocctine, viomycin, viquidil, visnadine, vitamin a derivatives, vitamin a, vitamin b2, vitamin d, vitamin e, vitamin k, voglibose, voriconazole, xaliproden, xamoterol, xanthinol niacinate, xenytropium bromide, xibenolol, ximelagatran, xylometazoline, yohimbine, zacopridc, zafirlukast, zafirlukat, zalcitabine, zaleplon, zanamivir, zatebradine, ziconotide, zidovudine, zileuton, zimeldine, zinc propionate, ziprasidone, zolimidine, zolmitriptan, zolpidem, zonisamide, zopiclone.

**B. SOLUBILIZERS**

In one embodiment, a pharmaceutical composition is provided comprising a therapeutically effective amount of an active agent, a solubilizer and a dispersing aid. The solubilizer is present in an amount such that more of the active agent is dispersed in aqueous medium than that which would be achieved with the same active agent and dispersing aid without the solubilizer. As mentioned above, the active agent and the solubilizer act synergistically to improve the dispersibility of the solubilizer itself and the active agent upon dilution in an aqueous media, thus greatly increasing the amount of active agent which can be dispersed in a readily absorbably form. Preferably, the solubilizer is present such that after a 100X dilution of the composition the active agent and/or the solubilizer is at least 30% dispersed in the aqueous phase, with a dispersion of at least 50% being preferred. It is more preferred that the solubilizer, like the active agent is at least 30% finely dispersed in the aqueous phase, with a fine dispersion of at least 50% being most preferred.

The preferred solubilizer of the present invention is a "vitamin E substance," which includes substances with the tocol structure [2-methyl-2-(4,8,12-trimethyltndecyl)chroman-6-ol] or the tocotrienol structure [2-methyl-2-(4,8,12-trimethyltrideca-3,7,11-trienyl)chroman-6-ol], in particular the all trans- (E,E) tocotrienols. Particularly preferred vitamin E substances include the mono-, di-, trimethyl- tocol derivatives, commonly known as tocopherols, such as α-tocopherol [5,7,8-trimethyl-], β-tocopherol [5,8-dimethyl-], γ-tocopherol [7,8-dimethyl], ζ₂-tocopherol [5,7-dimethyl-], δ-tocopherol [8-methyl-], η-tocopherol [7-methyl-]; and the corresponding mono-, di-, and trimethyltoctrienol derivatives, commonly known as tocotrienols, such as α-tocotrienol (or ζ₁-tocopherol) [5,7,8-trimethyl-], β-tocotricnol (or ε-tocopherol) [5,8-dimethyl-], γ-tocotrienol [7,8-dimethyl], and δ-tocotrienol [8-methyl-]. Included are their mixed racemic d1- forms, the pure d- and 1- enantiomers and the corresponding derivatives, e.g., esters, produced with organic acids; and mixtures thereof. Preferred vitamin E substances for use in the present invention include tocopherols, tocotrienols and tocopherol derivatives with organic acids such as acetic acid, propionic acid, bile acid, lactic acid, pyruvic acid, oxalic acid, malic acid, malonic acid, succinic acid, maleic acid, fumaric acid, tartaric acid, citric acid, benzoic acid, cinnamic acid, mandelic acid, polyethylene glycol succinate and salicylic acid. Particularly preferred vitamin E substances include alpha-tocopherol, alpha-tocopherol acetate, alpha-tocopherol acid succinate, alpha-tocopherol polyethylene glycol succinate and mixtures thereof.

Other solubilizers that may be used in the present invention arc disclosed in U.S. Patent Application Nos. 09/716,029 and 09/877,541, both to Chen et al. Preferred solubilizers that are not vitamin E substances for use in the present invention include fatty acid esters of glycerol, acetylated mono- and diglycerides, fatty acid esters of propylene glycol, trialkyl citrate, glycerol acetate, and lower alcohol fatty acid esters.

**C. SURFACTANTS**

The surfactant in the present invention may be any compound containing polar or charged hydrophilic moieties as well as non-polar hydrophobic (lipophilic) moieties; i.e. a surfactant compound must be amphiphilic. Within the context of the present invention, the hydrophilic surfactant can be any hydrophilic surfactant suitable for use in pharmaceutical compositions. Such surfactants can be anionic, cationic, zwitterionic or non-ionic. Mixtures of hydrophilic surfactants are also within the scope of the invention. Similarly, the hydrophobic surfactant can be any hydrophobic surfactant suitable for use in pharmaceutical compositions. Mixtures of hydrophobic surfactants are also within the scope of the invention. Generally, suitable hydrophilic surfactants will have an HLB value greater than about 10 and suitable hydrophobic surfactants will have an HLB value less than about 10. The choice of specific hydrophobic and hydrophilic surfactants should be made keeping in mind the particular hydrophobic therapeutic agent to be used in the composition, and the range of polarity appropriate for the chosen therapeutic agent. With these general principles in mind, a very broad range of surfactants is suitable for use in the present invention.

Examples of surfactants suitable for use in the present invention are disclosed in U.S. Patent No. 6,294,192 to Patel et al. and U.S. Patent Application No. 09/877,541 to Chen et al. Examples of surfactants that may be used in the present invention include polyethoxylated fatty acids such as PEG-8 laurate, PEG-8 oleate, PEG-8 stearate, PEG-9 oleate, PEG-10 laurate, PEG-10 oleate, PEG-12 laurate, PEG-12 oleate, PEG-15 oleate, PEG-20 laurate and PEG-20 oleate; PEG-fatty acid diesters such as PEG-20 dilaurate, PEG-20 dioleate, PEG-20 distearate, PEG-32 dilaurate and PEG-32 dioleate; PEG-fatty acid mono- and di-ester mixtures; polyethylene glycol glycerol fatty acid esters such as PEG-20 glyceryl laurate, PEG-30 glyceryl laurate, PEG-40 glyceryl laurate, PEG-20 glyceryl oleate, and PEG-30 glyceryl oleate; alcohol-oil transesterification products such as PEG-35 castor oil (Incrocas-35), PEG-40 hydrogenated castor oil (Cremophor® RH40), polyoxyl 35 castor oil (Cremophor EL), PEG-25 trioleate (TAGAT® TO), PEG-60 corn glycerides (Crovol M70), PEG-60 almond oil (Crovol A70), PEG-40 palm kernel oil (Crovol PK70), PEG-50 castor oil (Emalex C-50), PEG-50 hydrogenated castor oil (Emalex HC-50), PEG-8 caprylic/capric glycerides (Labrasol®), and PEG-6 caprylic/capric glycerides (Softigen® 767); transesterification products of oils and alcohols; polyglycerized fatty acids such as polyglyceryl oleate (Plurol® Oleique), polyglyccryl-2 dioleate (Nikkol DGDO), and polyglyceryl-10 trioleate. Preferred hydrophilic surfactants include polyglyceryl-10 laurate (Nikkol Decaglyn 1-L), polyglyceryl-10 oleate (Nikkol Dccaglyn 1-O), and polyglyceryl-10 mono, dioleate (Caprol® PEG 860); propylene glycol fatty acid esters such as propylene glycol monolaurate (Lauroglycol FCC), propylene glycol ricinoleate (Propymuls), propylene glycol monooleate (Myverol® P-06), propylene glycol dicaprylate/dicaprate (Captex® 200), and propylene glycol dioctanoate (Captex 800); mixtures of propylene glycol esters and glycerol esters such as a mixture of oleic acid esters of propylcnc glycol and glycerol (Arlacel 186); mono- and diglycerides such as glyceryl monooleate (Peceol), glyceryl ricinoleate, glyceryl laurate, glyceryl dilaurate (Capmul® GDL), glyceryl dioleate (Capmul GDO), glyceryl mono/dioleate (Capmul GMO-K), glyceryl caprylate/caprate (Capmul MCM), caprylic acid mono/diglycerides (Imwitor® 988), and mono- and diacetylated monoglycerides (Myvacet® 9-45); sterol and sterol derivatives such as PEG-24 cholesterol ether (Solulan® C-24); polyethylene glycol sorbitan fatty acid esters such as PEG-20 sorbitan monolaurate (Tween® 20), PEG-20 sorbitan monopalmitate (Tween 40), PEG-20 sorbitan monostearate (Tween 60), and PEG-20 sorbitan monooleate (polysorbate 80 or Tween 80); polyethylene glycol alkyl ethers such as PEG-3 oleyl ether (Volpo 3) and PEG-4 lauryl ether (Brij 30); sugar esters such as sucrose monopalmitate and sucrose monolaurate; polyethylene glycol alkyl phenols; polyoxyethylene-polyoxypropylene block copolymers such as Synperonic® PE series (ICI); Pluronie® series (BASF), Emkalyx, Lutrol (BASF), Supronic, Monolan, Pluracare®, and Plurodac; sorbitan fatty acid esters such as sorbitan monolaurate (Arlacel® 20), sorbitan monopalmitate (Span-40), sorbitan monooleate (Span-80), sorbitan monostearate, and sorbitan tristearate; lower alcohol fatty acid esters such as hydrophobic surfactants include ethyl oleate (Crodamol EO), isopropyl myristate (Crodamol IPM), and isopropyl palmitate (Crodamol IPP); ionic surfactants such as sodium oleate, sodium lauryl sulfate, sodium lauryl sarcosinate, sodium dioctyl sulfosuccinate, sodium cholate, sodium taurocholate, lauroyl carnitine, palmitoyl carnitine, and myristoyl carnitine; unionized ionizable surfactants such as free fatty acid, particularly C₆-C₂₂ fatty acids, and bile acids.

Other surfactants for use in the present invention include, without limitation, PEG-400 succinate, PEG 3350, tocopherol polyethyleneglycol (200-8000 MW) succinate, tocopherol polyethylene glycol 400 succinate, tocopherol polyethyleneglycol 1000 succinate (Vitamin E-TPGS, Eastman Chemical Co.), glycerol monolinoleate (Maisine®), propylene glycol monocaprylate (Capryol^{®} 90); caprylocaproyl macrogol-8 glycerides (Labrosol®), glycerol dibehenate (Compritol^{®} 888), glycerol distearate (Precirol®), lauroyl maarogol-32 glycerides (Gelueire® 44/14), and stearoyl macrogol-32 glycerides (Gelucire 50/13).

It is to be understood that within the context of the present invention, more than one solubilizer may be used. For example, ethanol may be used in conjunction with (Cremophor to improve the solubility of active agent. Preferred surfactants for use with particular active agents are illustrated in the Examples.

**D. OTHER ADDITIVES**

Although not always necessary, the compositions of the present invention may also include one or more additional components, i.e., additives. Classes of additives that may be present in the compositions, include, but are not limited to, solvents, absorbents, acids, adjuvants, anticaking agent, glidants, antitacking agents, antifoamers, anticoagulants, antimicrobials, antioxidants, antiphlogistics, astringents, antiseptics, bases, binders, chelating agents, sequestrants, coagulants, coating agents, colorants, dyes, pigments, compatiblizers, complexing agents, softeners, crystal growth regulators, denaturants, dessicants, drying agents, dehydrating agents, diluents, dispersants, emollients, emulsifiers, encapsulants, enzymes, fillers, extenders, flavor masking agents, flavorants, fragrances, gelling agents, hardeners, stiffening agents, humectants, lubricants, moisturizers, bufferants, pH control agents, plasticizers, soothing agents, demulcents, retarding agents, spreading agents, stabilizers, suspending agents, sweeteners, disintegrants, thickening agents, consistency regulators, surfactants, opacifiers, polymers, preservatives, antigellants, rheology control agents, UV absorbers, tonicifiers and viscomodulators. One or more additives from any particular class, as well as one or more different classes of additives, may be present in the compositions. Specific examples of additives are well known in the art.

**E. DOSAGE FORMS**

The pharmaceutical composition of the present invention can be prepared by mixing the active agent, the solubilizer, the surfactant, and optional additives according to methods well known in the art. Alternatively, the active agent, the solubilizer, and the surfactant may be prepared in separate dosage forms or separated within one dosage form to form a dispersion in situ upon administration and dissolution in the aqueous environment of the gastrointestinal tract.

The claimed pharmaceutical compositions can be further processed according to conventional methods known to those skilled in the art, such as lyophilization, encapsulation, compression, melting, extrusion, balling, drying, chilling, molding, spraying, spray congcaling, coating, comminution, mixing, homogenization, sonication, cryopelletization, spheronization and granulation to produce the desired dosage form. Excess solvent, added to facilitate incorporation of the active agent and/or mixing of the formulation components, can be removed before administration of the pharmaceutical dosage form.

The pharmaceutical compositions can be further formulated into desirable dosage forms utilizing skills well known in the art. For example, compositions in liquid, semisolid or paste form can be filled into hard gelatin or soft gelatin capsules using appropriate filling machines. Alternatively, the composition can also be extruded, merumerized, sprayed, granulated or coated onto a substrate to become a powder, granule or bead that can be further encapsulated or tableted with or without the addition of appropriate solidifying or binding agents. This approach also allows for the creation of a "fused mixture," a "solid solution" or a "eutectic mixture."

The dosage forms of the present invention are not limited with respect to size, shape or general configuration, and may comprise, for example, a capsule, a tablet or a caplct, or a plurality of granules, beads, powders, or pellets that may or may not be encapsulated. In addition, the dosage form may be a drink or beverage solution or a spray solution that is administered orally. Thus, for example, the drink or beverage solution may be formed by adding a therapeutically effective amount of the composition in, for example, a powder or liquid form, to a suitable beverage, e.g., water or juice.

The compositions and dosage forms of the current invention may be immediate release, releasing the active agent and/or excipients in an uncontrolled fashion, or may be controlled release. Included in the term "controlled release" are dosage forms or compositions which release the drug and/or excipients with various release profiles such as extended or sustained release, delayed release, pulsitile release, or combinations of the above such as multistage release achieved by a combination of delayed release compositions with variable delay times.

Preparation of various types of pharmaceutical formulations are described, for example, in Remington: The Science and Practice of Pharmacy, Nineteenth Edition. (1995) cited *supra* and Ansel et al., Pharmaceutical Dosage Forms and Drug Delivery Systems, 6th Ed. (Media, PA: Williams & Wilkins, 1995).

**II. UTILITY AND ADMINISTRATION**

The pharmaceutical compositions and dosage forms have utility in the treatment of patients that may benefit from the therapeutic administration of hydrophobic drugs. Such therapies include, for example, steroid therapy or hormone therapy. Patients suffering from any condition, disease or disorder that can be effectively treated with any of the active agents disclosed herein can benefit from the administration of a therapeutically effective amount of the pharmaceutical compositions and dosage forms described herein. An advantage of the claimed pharmaceutical composition is improvement in the oral absorption and bioavailability of the active agent thereby ensuring that the patient will in fact benefit from the prescribed therapy. The improved bioavailability of the active agent is a result of the improved dispersion of the active agent in the claimed pharmaceutical composition.

It is to be understood that while the invention has been described in conjunction with the preferred specific embodiments, the description set forth above as well as the examples that follow are intended only to illustrate the invention and not limit the scope of the invention. Other aspects, advantages, and modifications within the scope of the invention will be apparent to those skilled in the art to which the invention pertains.

All patents, patent applications, and publications mentioned herein, both *supra* and *infra*, are herein incorporated by reference.

### EXPERIMENTAL

The solubility of drug substances in the compositions was determined using conventional techniques. For example, solubility was in some cases determined gravimetrically by incrementally adding drug until the composition could no longer solubilize additional added drug. Solubility could also be determined by equilibration of the composition with excess drug during gentle mixing at a controlled temperature (25±0.5°C), centrifugation of the resulting mixture (15 min at 15,000*g; Beckmann Microfuge Lite), and assay of the clear supernatant.

The dispersibility of the composition was determined by diluting the composition in an aqueous medium such as water, simulated gastric fluid, or simulated intestinal fluid, at a selected dilution factor, preferably 10X to 1000X, most preferably 100X. The dilution was then gently mixed, for example with a rotator at 10 rpm, at an appropriate controlled temperature (typically 37°C). After a selected duration (typically 1 hour, but any physiologically realistic duration could be appropriate), the aqueous phase was sampled, taking care not to include undispersed oil globules, or non-uniformly dispersed particulates. In some cases, the aqueous phase was filtered through Nylon or Tuffryn^{®} membrane filters with the appropriate nominal pore size (Whatman or Gelman). In all cases, the initial 1-3 ml of filtrate were discarded, and the absence of significant filter absorption was confirmed by filtration of standard solutions of known active agent or vitamin E substance concentration in the appropriate matrix, collection of the filtrate, and assay of the filtrate to confirm that there was no change in drug concentration. Other techniques to characterize the extent of dispersion could also be used, such as centrifugation to separate larger particles from the uniform aqueous dispersion.

The aqueous phase sample or filtrate was then diluted in an appropriate solvent (typically acetonitrile or methanol; HPLC grade), and assayed for active agent or solubilizer content.

Assay for vitamin E substance content in most cases was by UV spectrophotometry with quantification at a wavelength of 291 nm for tocopherol and 285 nm for tocopherol acetate tocopherol succinate, and tocopherol polyethyleneglycol succinate. Samples were diluted 100X in methanol, then scanned in a quartz cuvette using an Agilent 8453 UV/Vis Spectrophotometer. Calibration was by linear regression of absorbance at the indicated wavelengths with standards of the relevant Vitamin E substance of known concentration. Standards of the drugs or other excipients present in the composition at the expected concentrations were also scanned to confirm selectivity.

In cases where the active agent or other excipient caused significant interference at the 285-291 nm wavelengths, assay for Vitamin E substances was by reversed phase HPLC using a Symmetry C18 3.6 X 150 mm column, 5 µ, with a mobile phase of Methanol 98/2%v/v and detection at 285 nm.

Assay of the active agents was by reversed-phase HPLC with the column indicated above, a mobile phase of acetonitrile/water 63/57%v/v, and detection at 204 nm.

Particle size of aqueous dispersions was determined using a Nicomp 380 ZLS laser-scattering particle sizer (Particle Sizing Systems), with a He-Ne laser at 632.8 nm, fixed 90° angle, interrupter at 13.5°, and maximum count rate 5 MHz.

The following Examples demonstrate the solubility characteristics of various embodiments of the claimed pharmaceutical formulation.

### EXAMPLE 1

This example shows the solubilization and dispersion behavior of a composition including a pregnane steroid, progesterone, a vitamin E substance (dl-alpha-tocopherol, Spectrum Chemicals) and a surfactant (polyoxyl 35 castor oil USP/NF, Cremophor EL, BASF). The compositions shown in the tables below were prepared by combining the components and mixing gently at room temperature.

**Table 1-1**

| **Component** | **Compositions** | | | |
|---|---|---|---|---|
| | **1-1** | **1-2** | **1-3** | **1-4** |
| dl-alpha tocopherol | 70% | 68.25% | 63% | 60% |
| Polyoxyl 35 Castor Oil | 30% | 29.25% | 27% | 26% |
| Progesterone | 0% | 2.5% | 10% | 15% |

Compositions were dispersed in simulated gastric fluid without enzyme (USP 23) at 100X dilution (37±0.5°C) and mixed gently for 1 hour. At 1 hour, the dispersions were filtered through 0.2 µ nominal pore size Nylon filters, then the filtrate was diluted 100X in methanol and assayed for progesterone by HPLC and for tocopherol content by UV/Vis spectrophotometry. Results are shown in Table 1-2 below

**Table 1-2**

| **No.** | **Drug Loading in Concentrate** | **Dispersion Appearance** | **Fraction of Solubilizer Dispersed** | **Fraction of Drug Dispersed** |
|---|---|---|---|---|
| 1-1 | 0 | Non-uniform with large oil globules and visible particulates | 14% | N/A |
| 1-2 | 25 mg/g | Non-uniform with large oil globules and visible particulates | 30% | 37% |
| 1-3 | 100 mg/g | Non-uniform with a few large globules | 41% | 36% |
| 1-4 | 150 mg/g | Uniform milky dispersion | 63% | 62% |

The results in Table 1-2 show that increasing the drug loading from 0 to 15% unexpectedly improves the dispersibility of the formulation. Without the drug, the composition does not disperse readily with most of the solubilizer present in separate oily globules. With the addition of the active agent, the dispersibility of the formulation is improved such that the fraction of drug dispersed significantly increases with increasing drug loading. The fraction of drug present as a very fine (<0.2 µ) dispersion increases from ∼37% at 25 mg/g drug loading to ∼62% at 15% drug loading. The improved dispersibility is also shown by the increase in the fraction of the solubilizer dispersed as a fine dispersion, increasing from 14% without drug to 63% with 150 mg/g drug.

### EXAMPLE 2

This example shows the solubilization and dispersion of a pregnane steroid, progesterone, in compositions consisting of vitamin E substances (dl-alpha-tocopherol, Spectrum Chemicals; or d-alpha-tocopherol, Archer Daniels Midland Company), a surfactant (polyoxyl 35 castor oil USP/NF, Cremophor EL, BASF), and a low-molecular weight alcohol (dehydrated alcohol, USP/NF, Quantum). The compositions shown in the tables below were prepared by combining the components and mixing gently at room temperature.

**Table 2-1**

| **Component** | **Compositions** | | | |
|---|---|---|---|---|
| | **2-1** | **2-2** | **2-3** | **2-4** |
| dl-alpha tocopherol | 65% | 54% | -- | -- |
| d-alpha tocopherol | -- | -- | 65% | 54% |
| Polyoxyl 35 Castor Oil | 28% | 23% | 28% | 23% |
| Ethanol | 7% | 6% | 7% | 6% |
| Progesterone | 0% | 17.5% | 0% | 17.5% |

Compositions were dispersed in simulated gastric fluid without enzyme (USP 23) at 100X dilution (37±0.5°C) and mixed gently for 1 hour. At 1 hour, the dispersions were filtered through 0.2 µ nominal pore size Nylon filters, then the filtrate was diluted 100X in methanol and assayed for tocopherol content by UV/Vis spectrophotometry and progesterone content by HPLC. The particle size distribution of the dispersions was independently determined by laser scattering with a Nicomp particle size analyzer for confirmation. Results are shown in Table 2-2 below

**Table 2-2**

| **No.** | **Vitamin E Substance** | **Drug** | **Dilution Appearance** | **Fraction of Solubilizer Dispersed in Filtrate <0.2 µ** | **Volume Fraction of Particles <0.2 µ, by laser scattering** | **Fraction of Drug Dispersed in Filtrate <0.2 µ** |
|---|---|---|---|---|---|---|
| 2-1 | dl-alpha-tocopherol | 0 mg/g | Non-uniform Large globules and particles in cloudy solution | 9% | N/A^{*} | -- |
| 2-2 | dl-alpha-tocopherol | 175 mg/g | Fine uniform dispersion | 66% | 79% | 100% |
| 2-3 | d-alpha-tocopherol | 0 mg/g | Non-uniform Large globules and particles in cloudy solution | 10% | N/A^{*} | -- |
| 2-4 | d-alpha-tocopherol | 175 mg/g | Fine uniform dispersion | 83% | 81% | 100% |

| | | | | | | |
|---|---|---|---|---|---|---|
| * Particle size cannot be accurately determined for non-uniform samples with very large particles. | | | | | | |

The results in Table 2-2 show that not only is the drug readily soluble in the vitamin E based composition, but the presence of the drug dramatically improves the dispersibility of the composition upon aqueous dilution. Without the drug, the composition does not form a fine dispersion and only ∼10% of the vitamin E is incorporated in particles <0.2 µ. With progesterone, the compositions form a very fine uniform dispersion, with ∼80% of the total vitamin E in particles <0.2 µ. The assay for progesterone in the filtered dispersions shows that the drug is preferentially concentrated in these very small particles with nominal diameter <0.2 µ.

### EXAMPLE 3

This example shows the solubilization and dispersion of an androstane steroid ((DHEA, Sigma Chemicals), in compositions consisting of vitamin E substances (dl-alpha-tocopherol, Spectrum Chemicals; or d-alpha-tocopherol, Archer Daniels Midland Company), a surfactant (polyoxyl 35 castor oil USP/NF, Cremophor EL, BASF), and a low-molecular weight alcohol (dehydrated alcohol, USP/NF, Quantum). The compositions shown in the tables below were prepared by combining the components and mixing gently at room temperature. The corresponding placeboes (without drug) are described in Example 2, compositions 2-1 and 2-3.

**Table 3-1**

| **Component** | **Compositions** | |
|---|---|---|
| | 3-1 | 3-2 |
| dl-alpha tocopherol | 54% | -- |
| d-alpha tocopherol | -- | 54% |
| Polyoxyl 35 Castor Oil | 23% | 23% |
| Ethanol | 6% | 6% |
| DHEA | 17.5% | 17.5% |

Compositions were dispersed in simulated gastric fluid without enzyme (USP 23) at 100X dilution (37±0.5°C) and mixed gently for 1 hour. At 1 hour, the dispersions were filtered through 0.2 µ nominal pore size Nylon filters, then the filtrate was diluted 100X in methanol and assayed for tocopherol content by UV/Vis spectrophotometry. Results are shown in Table 3-2 below

**Table 3-2**

| **No.** | **Vitamin E Substance** | **DHEA** | **Dilution Appearance** | **Fraction of Solubilizer** Dispersed |
|---|---|---|---|---|
| 2-1 | dl-alpha tocopherol | 0 mg/g | Non-uniform Complete phase separation with large oil globules | 9% |
| 3-1 | dl-alpha tocopherol | 175 mg/g | Fine, uniform dispersion | 68% |
| 2-3 | d-alpha tocopherol | 0 mg/g | Non-uniform Large globules in cloudy solution | 10% |
| 3-2 | d-alpha tocopherol | 175 mg/g | Fine, uniform dispersion | 70% |

The results in Table 3-2 show that, as with the pregnane steroid in example 2, the addition of the androstane steroid, dehydroepiandrosteroneDHEA, dramatically improves the formation of a fine dispersion of the composition resulting in compositions with very high drug loading, which are then readily dispersed in aqueous media.

### EXAMPLE 4

This example shows the solubilization and dispersion using Vitamin E/surfactant compositions for additional model steroids: an androstane steroid, finasteride; and a cholane steroid, ursodiol. The compositions shown in the tables below were prepared by combining the components and mixing gently at room temperature.

**Table 4-1**

| **Component** | **Compositions** | | |
|---|---|---|---|
| | **4-1** | **4-2** | **4-3** |
| dl-alpha tocopherol | 40.5% | 40% | 38% |
| Polyoxyl 35 Castor Oil | 49.5% | 49% | 46% |
| Ethanol | 10% | 5% | 5% |
| Finasteride | 0% | 1.1% | -- |
| Ursodiol | 0% | -- | 5.9% |

Compositions were dispersed in simulated gastric fluid without enzyme (USP 23) at 100X (37±0.5°C) and mixed gently for 1 hour. At 1 hour, the dispersions were filtered through 0.2 µ nominal pore size Nylon filters and the filtrate diluted 100X in methanol and assayed for tocopherol content by UV/Vis spectrophotometry. Results are shown in Table 4-2 below

**Table 4-2**

| **No.** | **Drug** | **Dilution Appearance** | **Fraction of Solubilizer Dispersed** |
|---|---|---|---|
| 4-1 | No drug | Non-uniform, large particles and globules | 40% |
| 4-2 | Finasteride | Fine, uniform dispersion | 86% |
| 4-3 | Ursodiol | Fine, uniform dispersion | 93% |

The results in Table 4-2 show that, as with the other steroids tested, the incorporation of the steroid active agent has a critical role in achieving good dispersion of the composition upon aqueous dilution.

### EXAMPLE 5

This example shows the solubilization and dispersion of progesterone in compositions containing two different tocopherol esters (d-alpha-tocopherol acetate and d-alpha-tocopherol succinate, Archer Daniels Midland Company). The compositions shown in the tables below were prepared by combining the components and mixing gently at room temperature.

**Table 5-1**

| **Component** | **Compositions** | | | |
|---|---|---|---|---|
| | **5-1** | **5-2** | **5-3** | **5-4** |
| d-alpha tocopherol acetate | 79% | 71% | -- | -- |
| d-alpha tocopherol succinate | -- | -- | 68% | 62% |
| Polyoxyl 35 Castor Oil | 14% | 13% | 29% | 27% |
| Ethanol | 7% | 6% | 3% | 3% |
| Progesterone | 0% | 10% | 0% | 8% |

Compositions were dispersed in simulated gastric fluid without enzyme (USP 23) at 100X dilution (37±0,5°C) and mixed gently for 1 hour. At 1 hour, the dispersions were filtered through 0.2 µ nominal pore size Nylon filters, then the filtrate was diluted 100X in methanol and assayed for tocopherol succinate content by UV/Vis spectrophotometry. Results are shown in Table 5-2 below

**Table 5-2**

| **No.** | **Vitamin E Substance** | **Drug** | **Dilution Appearance** | **Fraction of Solubilizer Dispersed** |
|---|---|---|---|---|
| 5-1 | d-alpha tocopherol acetate | 0 mg/g | Non-homogeneous dispersion, Large globules in cloudy solution | 28% |
| 5-2 | d-alpha tocopherol acetate | 100 mg/g | Fine uniform dispersion | 72% |
| 5-3 | d-alpha tocopherol succinate | 0 mg/g | Non-homogeneous dispersion, Large globules in cloudy solution | 66% |
| 5-4 | d-alpha tocopherol succinate | 84 mg/g | Fine uniform dispersion | 99% |

The results in Table 5-2 show that both tocopherol esters have excellent solubilizing capacity for the steroid and allow for very high drug loading. The results also show that the steroid is critical to achieving adequate dispersion of the composition. Without the steroid, the composition is visibly non-uniform with the bulk of the composition in large particles or globules (only 30% <0.45 µ). With the steroid drug, the composition is readily dispersed with more 70% of the particles in a fine dispersion which passes through the 0.45 µ filter.

### EXAMPLE 6

This example shows the effect of solubilization and dispersion of progesterone in compositions with varying surfactants and surfactant levels. The vitamin E substances are d-alpha tocopherol or d-alpha tocopherol acetate (both from Archer Daniels Midland) with the following surfactants: polyoxyl 35 castor oil (Cremophor EL, BASF); caprylocaproyl macrogolglycerides (Labrasol, Gattefosse); polysorbate 80 (Tween 80, ICI), medium chain monoglycerides (Capmul MCM, Abitec), and tocopherol polyethyleneglycol 1000 succinate (Vitamin E-TPGS, Eastman). The compositions shown in the tables below were prepared by combining the components and mixing gently at room temperature.

**Table 6-1**

| **Component** | **Tradename, Source** | **Composition** | | |
|---|---|---|---|---|
| | | **6-1** | **6-2** | **6-3** |
| dl-alpha tocopherol | Vitamin E USP, Spectrum | 77% | 29% | -- |
| dl-alpha tocopherol acetate | Vitamin E 6-100, ADM | -- | -- | 85% |
| Dehydrated Alcohol | Ethanol, 200 proof,Quantum | 15% | 3% | -- |
| Polyoxyl 35 Castor Oil | Cremophor EL, BASF | 9% | -- | -- |
| Caprylocaproyl macrogolglycerides | Labrasol,Gattefasse | -- | 68% | -- |
| Polysorbate 80 | Tween 80, ICI | -- | -- | 5% |
| Medium chain monoglycerides | Capmul MCM, Abitec | | | 7.5% |
| Tocopherol polyethylene glycol 1000 succinate | Vitamin E-TPGS, Eastman | -- | -- | 2,5% |
| Progesterone | N/A | 0% | 0% | 0% |

**Table 6-2**

| **Component** | **Tradename,Source** | **Composition** | | |
|---|---|---|---|---|
| | | **6-4** | **6-5** | **6-6** |
| dl-alpha tocopherol | Vitamin E USP, Spectrum | 59% | 27% | -- |
| dl-alpha tocopherol acetate | Vitamin E 6-100, ADM | -- | -- | 77.8% |
| Dehydrated Alcohol | Ethanol, Quantum | 7% | 3% | -- |
| Polyoxyl 35 Castor Oil | Cremophor EL, BASF | 12% | -- | -- |
| Caprylocaproyl macrogolglycerides | Labrasol,Gattefosse | -- | 63% | -- |
| Polysorbate 80 | Tween 80,ICI | -- | -- | 4.6% |
| Medium chain monoglycerides | Capmul MCM, Abitec | | | 6,9% |
| Tocopherol polyethylene glycol 1000 succinate | Vitamin E-TPGS, Eastman | -- | -- | 2.3% |
| Progesterone | N/A | 22.5% | 7% | 8.5% |

Compositions were dispersed in simulated gastric fluid without enzyme (USP 23) at 100X dilution (37±0.5°C) and mixed gently for 1 hour. At 1 hour, the bullc aqueous phase was sampled, taking care not to disturb the oily phase. The sample was then diluted 100X in methanol and assayed for tocopherol and drug content by UV/Vis spectrophotometry or HPLC. Results are shown in Table 6-3 below

**Table 6-3**

| **No.** | **Surfactant(s)** | **Vitamin E: Surfactant Ratio** | **Drug** | **Dilution Appearance** | **Fraction of Solubilizer Dispersed** | **Fraction of Drug Dispersed** |
|---|---|---|---|---|---|---|
| 6-1 | Polyoxyl 35 Castor Oil | 9:1 | 0 mg/g | Complete phase separation, visible oily layer, essentially clear aqueous phase | 0% | -- |
| 6-4 | Polyoxyl 35 Castor Oil | 9:1 | 225mg/g | Hazy dispersion with a few large visible globules | 69% | 65% |
| 6-2 | Caprylocaproyl macrogolglycerides | 3:7 | 0 mg/g | Complete phase separation with visible oily globules, cloudy aqueous phase | 17% | -- |
| 6-5 | Caprylocaproyl macrogolglycerides | 3:7 | 70 mg/g | Hazy dispersion with a few large visible globules | 68% | 69% |
| 6-3 | Polysorbate 80/Medium chain monoglycerides/ETPGS | 5.7:1 | 0 | Non uniform, cloudy with visible particulates | 44%^{a} | -- |
| 6-6 | Polysorbate 80/Medium chain monaglycerides/ETPGS | 5.7:1 | 85 mg/g | Completely dispersed in fine, slightly hazy dispersion | 100%^{a} | 100%^{a} |

| | | | | | | |
|---|---|---|---|---|---|---|
| a. Filtered with 0.45 µ filter before assay. | | | | | | |

The results in table 6-3 show that for all surfactants and surfactant levels, not only is the drug well solubilized in the vitamin E substance composition, but it also plays a critical role in dispersing the composition upon aqueous dilution.

### EXAMPLE 7

This example evaluates the dispersion behavior of an active agent, fenofibrate, in a composition of a tocopherol ester (d-alpha-tocopherol acetate, Archer Daniels Midland), and the surfactants, polysorbate 80 (Tween 80, ICI) and medium chain monoglycerides (Capmul MCM, Abitec). The compositions shown in the tables below were prepared by combining the components and mixing gently at room temperature.

**Table 7-1**

| **Component** | **Compositions** | |
|---|---|---|
| | **7-1** | **7-2** |
| d-alpha tocopherol acetate | 85% | 79% |
| Polysorbate 80 | 8.6% | 8% |
| Medium chain monoglycerides | 6.4% | 6% |
| Fenofibrate | 0% | 7% |

Compositions were dispersed in simulated gastric fluid without enzyme (USP 23) at 100X dilution (37±0.5°C) and mixed gently for 1 hour. At 1 hour, the dispersions were filtered through 0.2 µ nominal pore size Nylon filters, then the filtrate was diluted 100X in methanol and assayed for tocopherol acetate content by HPLC. Results are shown in Table 7-2 below

**Table 7-2**

| **No.** | **Drug Substance** | **Drug** | **Dilution Appearance** | **Fraction of Solubilizer Dispersed** |
|---|---|---|---|---|
| 7-1 | Placebo | 0 mg/g | Non-uniform Large globules in cloudy aqueous phase | 23% |
| 7-2 | Fenofibrate | 70 mg/g | Non-uniform Large globules in cloudy aqueous phase | 22% |

The results in Table 7-2 show that fenofibrate shows no synergism with the vitamin E substance solubilizer upon aqueous dilution and is not dispersed adequately in the aqueous medium for effective absorption.

### EXAMPLE 8

This example shows the effect of solubilization and dispersion of progesterone in a compositions consisting of a vitamin E substance (d-alpha-tocopherol), a surfactant (polyoxyl 35 castor oil USP/NF) and various hydrophilic and hydrophobic solvents (ethanol, triethyl citrate; glycerol triacetate (triacetin)). The compositions shown in the tables below were prepared by combining the components and mixing gently at room temperature.

**Table 8-1**

| **Component** | **Tradename, Source** | **Compositions** | | |
|---|---|---|---|---|
| | | **8-1** | **8-2** | **8-3** |
| dl-alpha tocopherol | Vitamin E USP, Spectrum | 65% | 65% | 65% |
| Polyoxyl 35 Oil Castor Oil | Cremophor EL, BASF 28% | 28% | 28% | 28% |
| Ethanol | Ethanol, 200 proof, Quantum | 7% | -- | -- |
| Triethyl citrate | Triethyl citrate, Aldrich | -- | 7% | -- |
| Triacetin | Triacetin, Eastman | -- | -- | 7% |
| Progesterone | N/A | 0% | 0% | 0% |

**Table 8-2**

| **Component** | **Tradename, Source** | **Compositions** | | |
|---|---|---|---|---|
| | | **8-4** | **8-5** | **8-6** |
| dl-alpha tocopherol | Vitamin E, USP, Spectrum | 59% | 59% | 59% |
| Polyoxyl 35 Castor Oil | Cremophor EL, BASF | 25% | 25% | 25% |
| Ethanol | Ethanol, 200 proof, Quantum | 6% | -- | -- |
| Triethyl citrate | Triethyl citrate, Aldrich | -- | 6% | -- |
| Triacetin | Triacetin, Eastman | -- | -- | 6% |
| Progesterone | N/A | 10% | 10% | 10% |

Compositions were dispersed in simulated gastric fluid without enzyme (USP 23) at 100X dilution (37±0.5°C) and mixed gently for 1 hour. At 1 hour, the dispersion were filtered through 0.2 µ nominal pore size Nylon filters, the filtrate was then diluted 100X in methanol and assayed for tocopherol content by UV/Vis spectrophotometry. Results are shown in Table 8-3 below.

**Table 8-2**

| **No.** | **Solvent** | **Drug** | **Dilution Appearance** | **Fraction of Solubilizer Dispersed** |
|---|---|---|---|---|
| 8-1 | Ethanol | 0 mg/g | Non uniform dispersion with large visible globules | 18% |
| 8-4 | Ethanol | 100 mg/g | Fine uniform dispersion | 69% |
| 8-2 | Triacetin | 0 mg/g | Non uniform dispersion with large visible globules | 24% |
| 8-5 | Triacetin | 100 mg/g | Fine uniform dispersion | 47% |
| 8-3 | Triethyl Citrate | 0 mg/g | Non uniform dispersion with large visible globules | 15% |
| 8-6 | Triethyl Citrate | 100 mg/g | Fine uniform dispersion | 45% |

This example shows that for each of the solvents tested, the presence of the steroid drug significantly improves the dispersibility of the composition in aqueous medium.

### EXAMPLE 9

This example shows the solubilization and dispersion of a water insoluble benzoquinone, Coenzyme Q10, in a composition consisting of a vitamin E substance (dl-alpha-tocopherol, BASF), and surfactant (Cremophor EL, BASF). Results are shown in Table 9-1. The corresponding composition without drug is in Example 1, Composition 1-1,

**Table 9-1**

| **Component** | **Composition** |
|---|---|
| | **9-1** |
| dl-alpha tocopherol | 63% |
| Cremophor EL | 27 |
| Coenzyme Q10 | 10% |

Compositions were dispersed in simulated gastric fluid without enzyme (USP 23) at 100X dilution (37±0.5°C) and mixed gently for 1 hour. At 1 hour, the aqueous phase was filtered through an 0.45 µ filter. The filtrate was then diluted 100X in methanol and assayed for tocopherol content by HPLC. Results are shown in Table 9-2 below.

**Table 9-2**

| **No.** | **Drug** | **Dilution Appearance** | **Fraction of Solubilizer Dispersed** |
|---|---|---|---|
| 1-1 | 0 mg/g | Non-uniform with large oil globules and visible particulates | 14% |
| 9-1 | 100 mg/g | Uniform, fine dispersion | 100% |

The results in Table 9-2 show that the benzoquinone, coenzyme Q10, synergistically improves the dispersion of the solubilizer. Without the active agent, the composition does not disperse in the aqueous environment (<14% of the solubilizer present as a fine dispersion <0.45 µ). With the active agent, the composition readily disperses to form a fine dispersion with 100% <0.45 µ.

### EXAMPLES 10-25

### Exemplary Compositions

### EXAMPLE 10

| **Component** | **Amount (mg)** |
|---|---|
| dl-alpha tocopherol | 520 |
| CremophorEL | 430 |
| DHEA | 50 |

### EXAMPLE 11

| **Component** | **Amount (mg)** |
|---|---|
| dl-alpha tocopherol | 55 |
| Cremophor RH40 | 45 |
| Dutasteride | 0.5 |

### EXAMPLE 12

| **Component** | **Amount (mg)** |
|---|---|
| dl-alpha tocopherol | 200 |
| Polysorbate 80 | 15 |
| Maisine (Glycerol | 30 |
| monolinoleate) | |
| Eplerenone | 40 |

### EXAMPLE 13

| **Component** | **Amount (mg)** |
|---|---|
| d1-alpha tocopherol | 300 |
| Capryol 90 (Propylene glycol monocaprylate) | 100 |
| Cremophor EL | 60 |
| Spironolactone | 200 |

### EXAMPLE 14

| **Component** | **Amount (mg)** |
|---|---|
| dl-alpha tocopherol | 313 |
| Cremophor EL | 256 |
| Dehydrated Alcohol | 70 |
| Progesterone | 60 |

### EXAMPLE 15

| **Component** | **Amount (mg)** |
|---|---|
| d-alpha tocopherol succinate | 60 |
| E-TPGS | 540 |
| PEG 8000 | 60 |
| Progesterone | 100 |

### EXAMPLE 16

| **Component** | **Amount (mg)** |
|---|---|
| d-alpha tocopherol succinate | 60 |
| E-TPGS | 540 |
| PEG 8000 | 60 |
| Testosterone | 100 |

### EXAMPLE 17

| **Component** | **Amount (mg)** |
|---|---|
| dl-alpha tocopherol | 300 |
| CremophorRH40 | 300 |
| Coenzyme Q10 | 100 |

### EXAMPLE 18

| **Component** | **Amount (mg)** |
|---|---|
| dl-alpha tocopherol | 300 |
| Cremophor RH40 | 300 |
| Idebenone | 90 |

### EXAMPLE 19

| **Component** | **Amount (mg)** |
|---|---|
| d-alpha tocopherol | 270 |
| Alpha-tocotrienol | 2 |
| Gamma-tocotrienol | 23 |
| Cremophor RH40 | 300 |
| Idebenone | 90 |

### EXAMPLE 20

| **Component** | **Amount (mg)** |
|---|---|
| dl-alpha tocopherol | 80 |
| Cremophor RH40 | 400 |
| Crovol M-40 | 350 |
| Coenzyme Q10 | 100 |

### EXAMPLE 21

| **Component** | **Amount (mg)** |
|---|---|
| Tocoperyl polyethylene glycol 400 succinate | 200 |
| Tocopherol polyethylene glycol 1000 succinate | 100 |
| PEG 3350 | 5 |
| Bicalutamide | 50 |

### EXAMPLE 22

| **Component** | **Amount (mg)** |
|---|---|
| d-alpha tocopherol succinate | 250 |
| Cremophor RH40 | 50 |
| Capmul MCM | 50 |
| Simvastatin | 10 |

### EXAMPLE 23

| **Component** | **Amount (mg)** |
|---|---|
| d-alpha tocopherol succinate | 200 |
| Cremophor RH40 | 200 |
| Glycerol Dibehenate (Compritol 888) | 100 |
| Glycerol Distearate (Precirol) | 80 |
| Metaxalone | 300 |

### EXAMPLE 24

| **Component** | **Amount (mg)** |
|---|---|
| d-alpha tocopherol succinate | 100 |
| Hydroxypropyl methyl cellulose, USP (Methocel K4M) | 100 |
| Microcrystalline cellulose, USP (Avicel PH 101) | 200 |
| Polyoxyl 40 Hydrogenated Castor Oil, USP (Cremophor RH 40) | 120 |
| Polyvinyl pyrrolidone, USP (Kollidon 90F) | 45 |
| Talc, USP | 8.75 |
| Colloidal Silicon dioxide, USP (Cab-o-Sil treated) | 1.25 |
| Dehydroepiandrosterone | 100 |

### EXAMPLE 25

| **Drug-Containing Granules:** | |
|---|---|
| **Component** | **Amount (mg)** |
| Spironolactone | 100.0 |
| Butylated Hydroxy Anisole USP-NF (BHA) | 0.05 |
| Microcrystalline Cellulose USP-NF | 100.0 |
| Crospovidone USP-NF | 27.5 |
| Polyvinyl pyrrolidone USP-NF | 40.0 |
| Talc USP-NF | 4.0 |
| Colloidal Silicon dioxide USP-NF | 2.0 |
| Magnesium Stearate USP-NF | 2.0 |

| **Solubilizer/Surfactant Granules: Component** | **Amount (mg)** |
|---|---|
| Cremophor RH40 | 300 |
| Tocopherol Polyethyleneglycol | 50 |
| 400 succinate | |
| d-alpha tocopherol succinate | 50 |
| Sodium Starch Glycolate USP-NF | 22 |
| Colloidal Silicon dioxide USP-NF | 122 |

The present invention will now be described by way of reference to the following clauses:
1. A pharmaceutical composition comprising:
   a. an active agent;
   b. a vitamin E substance; and
   c. a surfactant,
   wherein upon dilution of the composition, the active agent increases the extent of dispersion of the vitamin E substance by at least 20% relative to the dispersion of the composition without the active agent.
2. The pharmaceutical composition of clause 1, wherein the active agent is a hydrophobic drug.
3. The pharmaceutical composition of clause2, wherein the hydrophobic drug is a steroid.
4. The pharmaceutical composition of clause 2, wherein the hydrophobic drug is a benzoquinone.
5. The pharmaceutical composition of clause 1, wherein the vitamin E substance is selected from the group consisting of alpha tocopherol, alpha tocopherol acetate, alpha tocopherol succinate, and alpha tocopherol polyethyleneglycol succinate.
6. The pharmaceutical composition of clause 5, wherein the vitamin E substance is selected from the group consisting of d-alpha tocopherol, dl-alpha tocopherol, d-alpha tocopherol acetate, dl-alpha tocopherol acetate, d-alpha tocopherol succinate, and dl-alpha tocopherol succinate.
7. The pharmaceutical composition of clause 1, wherein the surfactant is selected from the group consisting of polyoxyl 35 castor oil, PEG-40 hydrogenated castor oil, caprylocaproyl macrogol-8 glycerides, polysorbate 80, lauroyl macrogol-32 glycerides, stearoyl macrogol-32 glycerides, and tocopherol polyethyleneglycol 1000 succinate.
8. A pharmaceutical composition comprising:
   a. a steroid;
   b. a vitamin E substance; and
   c. a surfactant,
   wherein after a 100X dilution of the composition in an aqueous medium, at least 30% of the hydrophobic drug or the vitamin E substance is dispersed in the aqueous phase.
9. The pharmaceutical composition according to clause 8, wherein at least 50% of the active agent or the vitamin E substance is dispersed in the aqueous phase.
10. The pharmaceutical composition according to clause 8, wherein at least 30% of the active agent or the vitamin E substance is finely dispersed in the aqueous phase.
11. The pharmaceutical composition according to clause 10, wherein at least 50% of the active agent or the vitamin E substance is finely dispersed in the aqueous phase.
12. The pharmaceutical composition of clause 8, wherein the steroid is present in an amount ranging from 0.01 % to 30% w/w of the composition, the vitamin E substance is present in an amount ranging from about 1% to 95% w/w of the composition, and the surfactant is present in an amount ranging from about 5 to 85% w/w of the composition.
13. The pharmaceutical composition of clause 12, wherein the steroid is progesterone.
14. The pharmaceutical composition of clause 12, wherein the steroid is testosterone.
15. The pharmaceutical composition of clause 12, wherein the steroid is dehydroepiantrosterone.
16. The pharmaceutical composition of clause 8, wherein the vitamin E substance is selected from the group consisting of alpha tocopherol, alpha tocopherol acetate, alpha tocopherol succinate, and alpha tocopherol polyethyleneglycol succinate.
17. The pharmaceutical composition of clause 16, wherein the vitamin E substance is selected from the group consisting of d-alpha tocopherol, dl-alpha tocopherol, d-alpha tocopherol acetate, dl-alpha tocopherol acetate, d-alpha tocopherol succinate, and dl-alpha tocopherol succinate.
18. The pharmaceutical composition of clause 8, wherein the surfactant is selected from the group consisting of polyoxyl 35 castor oil, PEG-40 hydrogenated castor oil, caprylocaproyl macrogol-8 glycerides, polysorbate 80, lauroyl macrogol-32 glycerides, stearoyl macrogol-32 glycerides, and tocopherol polyethyleneglycol 1000 succinate.
19. The pharmaceutical composition of clause 8, wherein the steroid is progesterone, the vitamin E substance is alpha tocopherol, and the surfactant is polyoxyl 35 castor oil.
20. The pharmaceutical composition of clause 19, wherein the progesterone is present in an amount ranging from about 0.1% to 30% w/w.
21. The pharmaceutical composition of clause 20, wherein the progesterone is present in an amount ranging from about 0.01% to 0.3% w/w.
22. The pharmaceutical composition of clause 8, wherein the steroid is dehydroepiantrosterone, the vitamin E substance is alpha tocopherol, and the surfactant is polyoxyl 35 castor oil.
23. The pharmaceutical composition of clause 22, wherein the dehydroepiantrosterone is present in an amount of at least 5% w/w.
24. The pharmaceutical composition of clause 8, wherein the steroid is testosterone, the vitamin E substance is alpha tocopherol succinate, and the surfactant is tocopherol polyethyleneglycol 1000 succinate.
25. The pharmaceutical composition of clause 24, wherein the testosterone is present in an amount of at least 1% w/w.
26. The pharmaceutical composition of clause 8, wherein the steroid is progesterone, the vitamin E substance is alpha tocopherol succinate, and the surfactant is tocopherol polyethyleneglycol 1000 succinate.
27. The pharmaceutical composition of clause 26, wherein the progesterone is present in an amount ranging from about 1% to 30%.
28. A method of improving the bioavailability of a hydrophobic drug administered to a patient comprising administering to said patient a therapeutically effective amount of the pharmaceutical composition of clause 2 in a suitable dosage form.
29. A method of improving the bioavailability of a steroid administered to a patient comprising administering to said patient a therapeutically effective amount of the pharmaceutical composition of clause 8 in a suitable dosage form.

## Claims

1. A pharmaceutical composition comprising:
a. an active agent;
b. a vitamin E substance; and
c. a surfactant,
wherein upon dilution of the composition, the active agent increases the extent of dispersion of the vitamin E substance by at least 20% relative to the dispersion of the composition without the active agent, wherein the active agent is testosterone, or an ester thereof.

2. The pharmaceutical composition of claim 1, wherein the surfactant is selected from the group consisting of polyoxyl 35 castor oil, PEG-40 hydrogenated castor oil, caprylocaproyl macrogol-8 glycerides, polysorbate 80, lauroyl macrogol032 glycerides, stearoyl macrogol-32 glycerides, and tocopherol polyethyleneglycol 1000 succinate.

3. A pharmaceutical composition comprising:
a. a steroid;
b. a vitamin E substance; and
c. a surfactant,
wherein after a 100X dilution of the composition in an aqueous medium, at least 30% of the hydrophobic drug or the vitamin E substance is dispersed in the aqueous phase, wherein the steroid is testosterone or an ester thereof.

4. The pharmaceutical composition according to claim 3, wherein at least 50% of the active agent or the vitamin E substance is dispersed in the aqueous phase.

5. The pharmaceutical composition according to claim 3, wherein at least 30% of the active agent or the vitamin E substance is finely dispersed in the aqueous phase.

6. The pharmaceutical composition according to claim 5, wherein at least 50% of the active agent or the vitamin E substance is finely dispersed in the aqueous phase.

7. The pharmaceutical composition of claim 3, wherein the steroid is present in an amount ranging from 0.01% to 30% w/w of the compostion, the vitamin E substance is present in an amount ranging from about 1% to 95% w/w of the composition, and the surfactant is present in an amount ranging from about 5 to 85% w/w of the composition.

8. The pharmaceutical composition of claim 1 or 3, wherein the vitamin E substance is selected from the group consisting of alpha tocopherol, alpha tocopherol acetate, alpha tocopherol succinate, and alpha tocopherol polyethyleneglycol succinate.

9. The pharmaceutical composition of claim 8, wherein the vitamin E substance is selected from the group consisting of d-alpha tocopherol, dl-alpha tocopherol, d-alpha tocopherol acetate, dl-alpha tocopherol acetate, d-alpha tocopherol succinate, and dl-alpha tocopherol succinate.

10. The pharmaceutical composition of claim 3, wherein the surfactant is selected from the group consisting of polyoxyl 35 castor oil, PEG-40 hydrogenated castor oil, caprylocaproyl macrogol-8 glycerides, polysorbate 80, lauroyl macrogol-32 glycerides, stearoyl macrogol-32 glycerides, and tocopherol polyethyleneglycol 1000 succinate.

11. The pharmaceutical composition of claim 3, wherein the steroid is testosterone, the vitamin E substance is alpha tocopherol succinate, and the surfactant is tocopherol polyethyleneglycol 1000 succinate.

12. The pharmaceutical composition of claim 3, wherein the testosterone is present in an amount of at least 1% w/w.

13. The composition of claim 1 for use in a method of improving the biovailability of a hydrophobic drug administered to a patient, the method comprising administering to said patient a therapeutically effective amount of the pharmaceutical composition of claim 1 in a suitable dosage form.

14. The composition of claim 3 for use in a method of improving the bioavailability of a steroid administered to a patient, the method comprising administering to said patient a therapeutically effective amount of the pharmaceutical composition of claim 3 in a suitable dosage form.
